# EUROPEAN PATENT SPECIFICATION

(11) **EP 3 777 691 B1**
(45) Date of publication and mention of the grant of the patent: **20.03.2024**
(21) Application number: 20190788.8
(22) Date of filing: 12.08.2020
(51) Int. Cl.: A61B 6/02, A61B 6/00, G16H 30/00, G16H 50/20, G16H 30/40, G16H 30/20, A61B 6/04

(54) **IMAGE DISPLAY DEVICE, IMAGE DISPLAY METHOD, IMAGE DISPLAY PROGRAM, IMAGE MANAGEMENT DEVICE, IMAGE MANAGEMENT METHOD, AND IMAGE MANAGEMENT PROGRAM**
BILDANZEIGEVORRICHTUNG, BILDANZEIGEVERFAHREN, BILDANZEIGEPROGRAMM, BILDVERWALTUNGSVORRICHTUNG, BILDVERWALTUNGSVERFAHREN UND BILDVERWALTUNGSPROGRAMM
DISPOSITIF D'AFFICHAGE D'IMAGES, PROCÉDÉ D'AFFICHAGE D'IMAGES, PROGRAMME D'AFFICHAGE D'IMAGES, DISPOSITIF DE GESTION D'IMAGES, PROCÉDÉ DE GESTION D'IMAGES ET PROGRAMME DE GESTION D'IMAGES

(30) Priority: 15.08.2019 JP 2019149104
(43) Date of publication of application: 17.02.2021
(73) Proprietor: FUJIFILM Corporation, Tokyo 106-8620 (JP)
(72) Inventor: FUKUDA, Wataru, Ashigarakami-gun, Kanagawa 258-8538 (JP)
(74) Representative: Dewhurst, Joseph Michael

(56) References cited:
- EP-A2- 3 785 635
- JP-A- 2013 075 065
- US-A1- 2004 114 714
- US-A1- 2009 123 052
- US-A1- 2012 301 003
- US-A1- 2016 015 333
- US-A1- 2016 089 099

## Description

### BACKGROUND

### Technical Field

The present disclosure relates to an image display device, an image display method, an image display program, an image management device, an image management method, and an image management program that display tomographic images acquired by tomosynthesis imaging.

### Related Art

In recent years, image diagnosis using a radiography apparatus (called mammography) for capturing an image of the breast has attracted attention in order to promote early detection of breast cancer. Further, in the mammography, tomosynthesis imaging has been proposed which moves a radiation source, irradiates the breast with radiation from a plurality of radiation source positions to acquire a plurality of projection images, adds the plurality of acquired projection images to generate tomographic images in which desired tomographic planes have been highlighted. In the tomosynthesis imaging, the radiation source is moved in parallel to a radiation detector or is moved so as to draw a circular or elliptical arc according to the characteristics of an imaging apparatus and the required tomographic image and imaging is performed for the breast at a plurality of radiation source positions to acquire a plurality of projection images. Then, the projection images are reconstructed using, for example, a back projection method, such as a simple back projection method or a filtered back projection method, or a sequential reconstruction method to generate tomographic images.

The tomographic images are generated in a plurality of tomographic planes of the breast, which makes it possible to separate structures that overlap each other in the depth direction in which the tomographic planes are arranged in the breast. Therefore, it is possible to find an abnormal part such as a lesion that has been difficult to detect in a two-dimensional image (hereinafter, referred to as a simple two-dimensional image) acquired by simple imaging according to the related art.

In addition, a technique has been known which combines a plurality of tomographic images having different distances (positions in a height direction) from a detection surface of a radiation detector to a radiation source, which have been acquired by tomosynthesis imaging, using, for example, an addition method, an averaging method, a maximum intensity projection method, or a minimum intensity projection method to generate a pseudo two-dimensional image (hereinafter, referred to as a composite two-dimensional image) corresponding to the simple two-dimensional image (see JP2014-128716A). In the composite two-dimensional image, an abnormal part included in the tomographic image is less affected by the tissues in the thickness direction of the breast than that in the simple two-dimensional image. Therefore, the use of the composite two-dimensional image makes it easy to interpret an abnormal part in the breast with one image.

In contrast, in the medical field, a computer aided diagnosis (hereinafter, referred to as CAD) system has been known which automatically detects an abnormal shadow, such as a lesion, in an image and displays the detected abnormal shadow so as to be highlighted. For example, the CAD is used to detect important structures in diagnosis, such as calcifications, spicula, and tumor, from the tomographic images acquired by the tomosynthesis imaging. In addition, a method has been proposed which, in a case in which a composite two-dimensional image is generated from a plurality of tomographic images acquired by performing the tomosynthesis imaging for the breast, detects a region of interest including an abnormal part using the CAD and combines the detected region of interest on the composite two-dimensional image (see US8983156B).

The tomosynthesis imaging is performed to generate a plurality of tomographic images and a composite two-dimensional image. However, as described above, in a case in which the number of types of images used for diagnosis increases, the image storage capacity of an image archiving and communication system (PACS) that stores a plurality of images for diagnosis increases. In contrast, the tomosynthesis imaging is performed to generate a large number of images. However, in the actual diagnosis, it may be sufficient to interpret a composite two-dimensional image or one of a plurality of projection images. In addition, it may be sufficient to interpret only the tomographic image in which an abnormal part has been detected by the CAD.

Therefore, a method has been proposed which selects a key tomographic image, such as a tomographic image in which an abnormal part has been detected, from a plurality of tomographic images and stores the selected tomographic image in the PACS. For example, a method has been proposed which receives the designation of a region of interest including an abnormal part in a plurality of tomographic images and stores only a tomographic image including the region of interest in the PACS (see JP2013-075065A). In a case in which only the tomographic image in which an abnormal part is detected by CAD is stored in the PACS, it is possible to reduce the image storage capacity.

However, in the method described in JP2013-075065A, the operator needs to set the key image to be stored. Therefore, a burden on the operator is large. Further, the accuracy of the detection result of an abnormal part by the CAD is not always high. Therefore, in a case in which only the tomographic image in which an abnormal part has been detected by the CAD is stored, a situation occurs in which a tomographic image including an abnormal part is not actually stored.

US2004114714 discloses a CAD system.

### SUMMARY OF THE INVENTION

The present disclosure has been made in view of the above-mentioned problems and an object of the present disclosure is to provide a technique that can appropriately reduce the capacity of an image acquired by tomosynthesis imaging.

According to the present disclosure, there is provided an image display device as claimed in claim 1.

The image display device according to the present disclosure may further comprise an abnormal part detection unit that detects an abnormal part from the tomographic image. The display control unit may display a region of interest including the abnormal part on the display unit in a case in which a tomographic image in which the abnormal part has been detected by the abnormal part detection unit is displayed.

In the image display device according to the present disclosure, the display control unit may display a composite two-dimensional image generated by combining the plurality of tomographic images on the display unit and may display a tomographic image including an abnormal part designated in the composite two-dimensional image on the display unit.

In the image display device according to the present disclosure, in a case in which the tomosynthesis imaging is performed for the object in a plurality of directions and one of the plurality of tomographic images is displayed on the display unit for any one of the plurality of imaging directions, the setting unit may set all of the tomographic images in the imaging direction as having been displayed.

In the image display device according to the present disclosure, the setting unit may further set at least one tomographic image in a tomographic plane which is adjacent to the displayed tomographic image as having been displayed.

In the image display device according to the present disclosure, the setting unit may change the tomographic image set as having been displayed to a non-display state in response to a command from an operator.

In the image display device according to the present disclosure, the object may be a breast.

A first image management device according to the present disclosure may comprise: a storage unit that stores a plurality of tomographic images acquired by performing tomosynthesis imaging for an object; and a storage control unit that deletes tomographic images other than the tomographic image set as having been displayed from the storage unit on the basis of a setting result of the setting unit in at least one image display device according to the present disclosure.

In the first image management device according to the present disclosure, the storage control unit may delete the other tomographic images from the storage unit on the basis of the setting result of the setting unit in at least one of a plurality of the image display devices.

A second image management device according to the present disclosure may comprise: a storage unit that stores a plurality of tomographic images acquired by performing tomosynthesis imaging for an object; and a storage control unit that sets a compression rate of tomographic images other than the tomographic image set as having been displayed to be higher than a compression rate of the tomographic image set as having been displayed on the basis of a setting result of the setting unit in at least one image display device according to the present disclosure and stores the plurality of tomographic images in the storage unit.

In the second image management device according to the present disclosure, the storage control unit sets the compression rate of the other tomographic images to be higher than the compression rate of the tomographic image set as having been displayed on the basis of a setting result of the setting unit in at least one of a plurality of the image display devices and may store the plurality of tomographic images in the storage unit.

According to the present disclosure, there is provided an image display method as claimed in claim 11.

A first image management method according to the present disclosure comprises: storing a plurality of tomographic images acquired by performing tomosynthesis imaging for an object in a storage unit; and deleting tomographic images other than the tomographic image set as having been displayed from the storage unit on the basis of a setting result of the setting unit in at least one image display device according to the present disclosure.

A second image management method according to the present disclosure comprises: storing a plurality of tomographic images acquired by performing tomosynthesis imaging for an object in a storage unit; and setting a compression rate of tomographic images other than the tomographic image set as having been displayed to be higher than a compression rate of the tomographic image set as having been displayed on the basis of a setting result of the setting unit in at least one image display device according to the present disclosure and storing the plurality of tomographic images in the storage unit.

In addition, programs that cause a computer to perform the image display method and the first and second image management methods according to the present disclosure may be provided.

The image display device may comprise a memory that stores commands to be executed by a computer and a processor configured to execute the stored commands. The processor performs a process of displaying, on a display unit, a tomographic image for which a display command is given among a plurality of tomographic images acquired by performing tomosynthesis imaging for an object and a process of setting the displayed tomographic image as having been displayed.

The image management device may comprise a memory that stores commands to be executed by a computer and a processor configured to execute the stored commands. The processor performs a process of storing a plurality of tomographic images acquired by performing tomosynthesis imaging for an object in a storage unit and a process of deleting tomographic images other than the tomographic image set as having been displayed from the storage unit on the basis of a setting result of the setting unit in at least one image display device according to the present disclosure.

The image management device may comprise a memory that stores commands to be executed by a computer and a processor configured to execute the stored commands. The processor performs a process of storing a plurality of tomographic images acquired by performing tomosynthesis imaging for an object in a storage unit and a process of setting a compression rate of tomographic images other than the tomographic image set as having been displayed to be higher than a compression rate of the tomographic image set as having been displayed on the basis of a setting result of the setting unit in at least one image display device according to the present disclosure and storing the plurality of tomographic images in the storage unit.

According to the present disclosure, it is possible to appropriately reduce the capacity of a tomographic image obtained by tomosynthesis imaging.

### BRIEF DESCRIPTION OF THE DRAWINGS

Fig. 1 is a diagram schematically illustrating a configuration of a radiographic image interpretation system to which an image display device and an image management device according to an embodiment of the present disclosure are applied.
Fig. 2 is a diagram schematically illustrating a configuration of a radiography system.
Fig. 3 is a diagram illustrating a mammography apparatus as viewed from a direction of an arrow A in Fig. 2.
Fig. 4 is a diagram schematically illustrating a configuration of an imaging control device implemented by installing an imaging program in a computer forming a console.
Fig. 5 is a diagram illustrating the acquisition of projection images.
Fig. 6 is a diagram illustrating the generation of tomographic images.
Fig. 7 is a diagram schematically illustrating a configuration of the image display device implemented by installing an image display program in the computer.
Fig. 8 is a diagram illustrating association between a lesion and a tomographic image.
Fig. 9 is a diagram illustrating a composite two-dimensional image display screen.
Fig. 10 is a diagram illustrating a display screen on which a tomographic image is displayed in addition to a composite two-dimensional image.
Fig. 11 is a diagram illustrating a state in which a mark is added to a displayed tomographic image.
Fig. 12 is a diagram illustrating a display screen on which a display change button is displayed.
Fig. 13 is a diagram illustrating setting information.
Fig. 14 is a diagram illustrating the deletion of tomographic images other than the displayed tomographic images.
Fig. 15 is a diagram illustrating a confirmation screen for deleting other tomographic images.
Fig. 16 is a diagram schematically illustrating a configuration of the image management device implemented by installing an image management program in the computer.
Fig. 17 is a flowchart illustrating a process performed in the image display device according to this embodiment.
Fig. 18 is a flowchart illustrating a process performed in the image management device according to this embodiment.
Fig. 19 is a diagram illustrating a confirmation screen for permitting whether or not to transmit the setting information.
Fig. 20 is a diagram illustrating tomographic images in tomographic planes adjacent to a displayed tomographic image.
Fig. 21 is a diagram illustrating tomographic images displayed by a plurality of doctors.
Fig. 22 is a diagram illustrating another example of the display screen.
Fig. 23 is a diagram illustrating still another example of the display screen.
Fig. 24 is a diagram illustrating yet another example of the display screen.
Fig. 25 is a diagram illustrating still yet another example of the display screen.

### DETALED DESCRIPTION

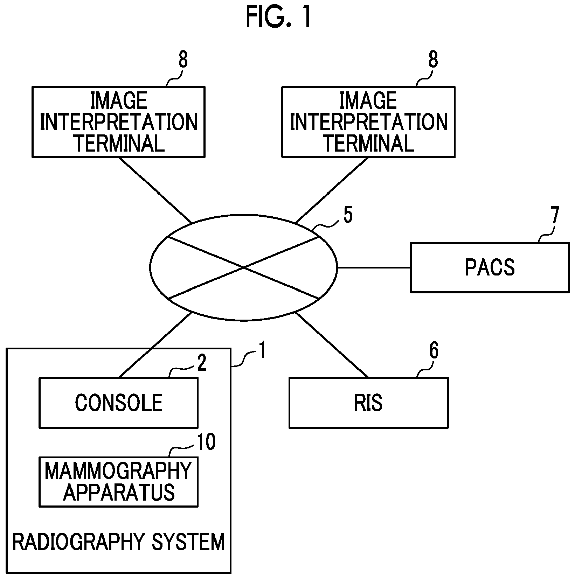

Hereinafter, embodiments of the present disclosure will be described with reference to the drawings. Fig. 1 is a diagram schematically illustrating a configuration of a radiographic image interpretation system to which an image display device and an image management device according to an embodiment of the present disclosure are applied. As illustrated in Fig. 1, in the radiographic image interpretation system according to this embodiment, a radiography system 1 including a console 2 and a mammography apparatus 10, a radiology information system (RIS) 6, a picture archiving and communication system (PACS) 7, and a plurality of image interpretation terminals (two image interpretation terminals in Fig. 1) 8 are connected through a network 5 so as to communicate with each other.

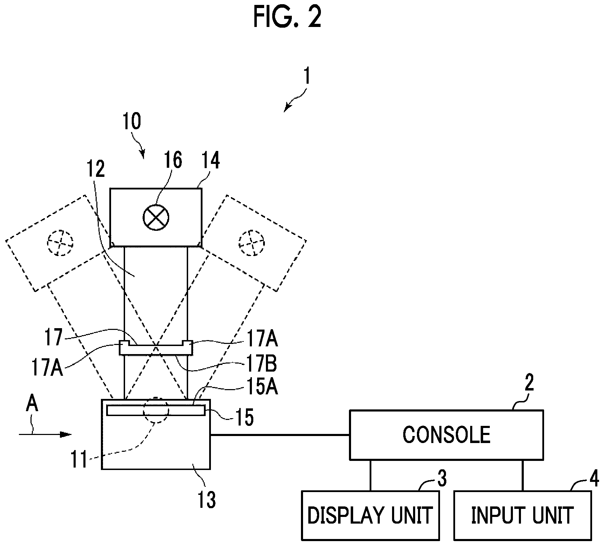

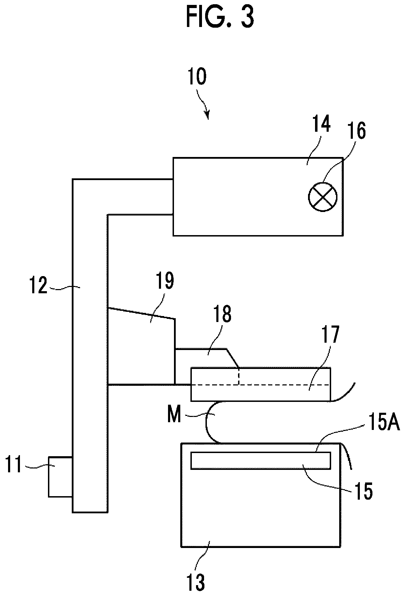

Fig. 2 is a diagram schematically illustrating a configuration of the radiography system and Fig. 3 is a diagram illustrating the mammography apparatus included in the radiography system as viewed from the direction of an arrow A in Fig. 2.

As illustrated in Fig. 2, the radiography system 1 includes the console 2 and the mammography apparatus 10. The console 2 comprises a display unit 3 and an input unit 4. The console 2 is connected to the RIS 6 and the PACS 7 through the network 5 so as to communicate therewith.

The radiography system 1 according to this embodiment has a function of capturing the images of a breast M using the mammography apparatus 10 on the basis of a command (imaging order) input from the RIS 6 through the console 2 in response to an operation of an operator, such as a doctor or a radiology technician, and acquiring a tomographic image and a composite two-dimensional image of the breast M. In this embodiment, the mammography apparatus 10 can perform both tomosynthesis imaging and simple imaging in various imaging directions to generate a tomographic image and a two-dimensional breast image of the breast M. The two-dimensional breast image means a breast image acquired by the simple imaging. In this embodiment, the description will be made assuming that the simple imaging is not performed and only the tomosynthesis imaging is performed. An image set including the tomographic image and the composite two-dimensional image generated in the radiography system 1 as described below is transmitted to the PACS 7 and is then stored therein.

The mammography apparatus 10 comprises an arm portion 12 that is connected to a base (not illustrated) by a rotation shaft 11. An imaging table 13 is attached to one end of the arm portion 12 and a radiation emitting unit 14 is attached to the other end of the arm portion 12 so as to face the imaging table 13. The arm portion 12 is configured such that only the end to which the radiation emitting unit 14 is attached can be rotated. Therefore, the imaging table 13 is fixed and only the radiation emitting unit 14 can be rotated. The rotation of the arm portion 12 is controlled by the console 2.

A radiation detector 15, such as a flat panel detector, is provided in the imaging table 13. The radiation detector 15 has a radiation detection surface 15A. In addition, for example, a circuit substrate including a charge amplifier that converts a charge signal read from the radiation detector 15 into a voltage signal, a correlated double sampling circuit that samples the voltage signal output from the charge amplifier, and an analog-digital (AD) conversion unit that converts the voltage signal into a digital signal is provided in the imaging table 13.

The radiation detector 15 can repeatedly perform the recording and reading of a radiographic image and may be a so-called direct-type radiation detector that directly converts radiation into charge or a so-called indirect-type radiation detector that converts radiation into visible light once and converts the visible light into a charge signal. As a method for reading a radiographic image signal, it is desirable to use the following method: a so-called thin film transistor (TFT) reading method which turns on and off a TFT switch to read a radiographic image signal; or a so-called optical reading method which emits reading light to read a radiographic image signal. However, the reading method is not limited thereto and other methods may be used.

A radiation source 16 is accommodated in the radiation emitting unit 14. The radiation source 16 emits, for example, X-rays as radiation. The console 2 controls the timing when the radiation source 16 emits the radiation and the radiation generation conditions of the radiation source 16, that is, the selection of target and filter materials, a tube voltage, an irradiation time, and the like.

Further, the arm portion 12 is provided with a compression plate 17 that presses and compresses the breast M, a support portion 18 that supports the compression plate 17, and a movement mechanism 19 that moves the support portion 18 in the vertical direction in Figs. 2 and 3. An interval between the compression plate 17 and the imaging table 13, that is, a compression thickness is input to the console 2. In addition, the compression plates 17 having a plurality of sizes and shapes corresponding to the types of imaging are prepared. Therefore, the compression plate 17 is attached to the support portion 18 so as to be interchangeable. Further, side walls 17A are formed on the left and right edges of the compression plate 17 in Fig. 2. The side walls 17A are formed in order to reduce the pain of a patient in a case in which the breast M compressed by a compression surface 17B of the compression plate 17 protrudes from the compression plate 17.

The display unit 3 is a display, such as a cathode ray tube (CRT) or a liquid crystal display, and displays messages required for operations in addition to a tomographic image and a composite two-dimensional image which will be described below. The display unit 3 may include a speaker that outputs sound.

The input unit 4 consists of a keyboard, a mouse, or a touch-panel-type input device and receives commands to operate the mammography apparatus 10 from the operator. In addition, the input unit 4 receives the input of various kinds of information required for tomosynthesis imaging, such as imaging conditions, and a command to correct information. In this embodiment, each unit of the mammography apparatus 10 is operated according to the information input by the operator through the input unit 4.

An imaging program for performing, for example, tomosynthesis imaging is installed in the console 2. In this embodiment, the console 2 may be a workstation or a personal computer that is directly operated by the operator or a server computer that is connected to them through a network. The imaging program is stored in a storage device of a server computer connected to the network or a network storage in a state in which it can be accessed from the outside and is downloaded and installed in the computer as required. Alternatively, the imaging program is recorded on a recording medium, such as a digital versatile disc (DVD) or a compact disc read only memory (CD-ROM), is distributed, and is installed in a computer from the recording medium.

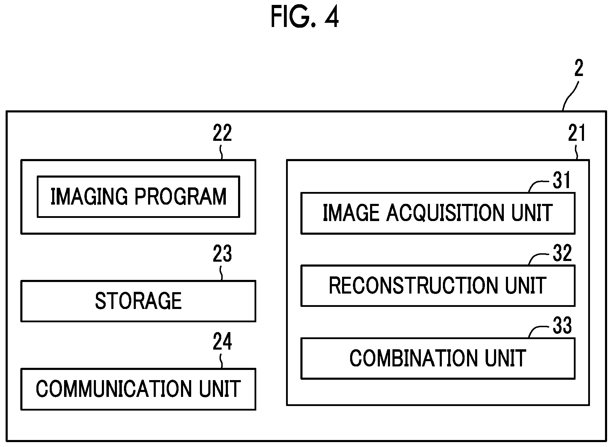

Fig. 4 is a diagram schematically illustrating a configuration of an imaging control device that is implemented by installing the imaging program in a computer forming the console 2. As illustrated in Fig. 4, the imaging control device comprises a central processing unit (CPU) 21, a memory 22, a storage 23, and a communication unit 24 as a standard computer configuration.

The storage 23 is a storage device, such as a hard disk drive or a solid state drive (SSD), and stores various kinds of information including an imaging program for driving each unit of the mammography apparatus 10 to perform tomosynthesis imaging. Further, for example, a projection image acquired by imaging, and tomographic images and a composite two-dimensional image generated as described below are stored in the storage 23.

The communication unit 24 is a network interface that controls the transmission of various kinds of information through the network 5.

The memory 22 temporarily stores, for example, the programs that are stored in the storage 23 in order to cause the CPU 21 to perform various processes. The imaging program defines the following processes as the processes to be performed by the CPU 21: an image acquisition process that causes the mammography apparatus 10 to perform tomosynthesis imaging to acquire a plurality of projection images of the breast M corresponding to each of a plurality of radiation source positions; a reconstruction process that reconstructs the plurality of projection images to generate a plurality of tomographic images in each of a plurality of tomographic planes of the breast M as an object; and a combination process that generates a composite two-dimensional image from the plurality of tomographic images.

The CPU 21 of the console 2 performs these processes according to the imaging program such that the CPU 21 functions as an image acquisition unit 31, a reconstruction unit 32, and a combination unit 33.

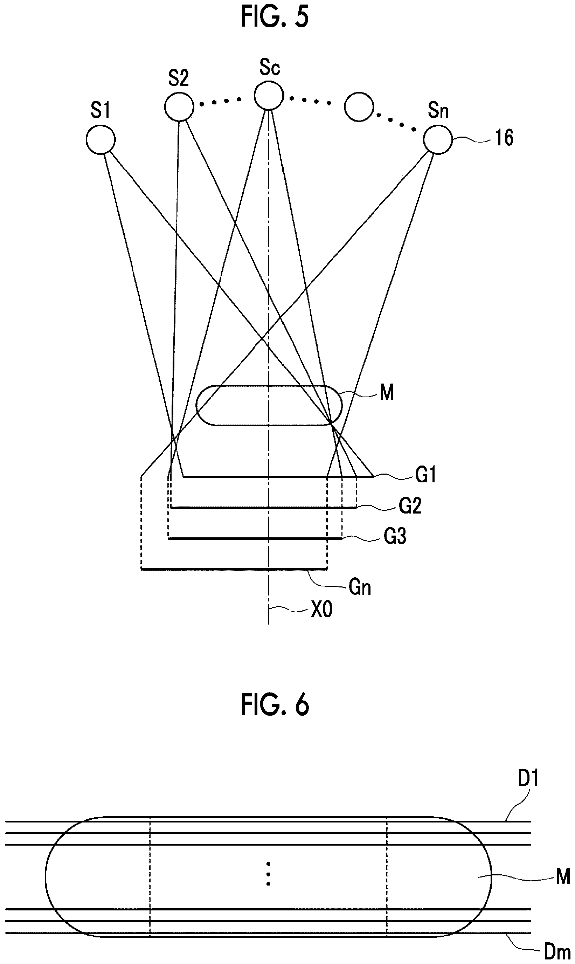

The image acquisition unit 31 rotates the arm portion 12 around the rotation shaft 11 to move the radiation source 16, irradiates the breast M with radiation at a plurality of radiation source positions caused by the movement of the radiation source 16 according to imaging conditions for tomosynthesis imaging, detects the radiation transmitted through the breast M using the radiation detector 15, and acquires a plurality of projection images Gi (i = 1 to n, where n is the number of radiation source positions and is, for example, 15) at a plurality of radiation source positions. Fig. 5 is a diagram illustrating the acquisition of the projection images Gi. As illustrated in Fig. 5, the radiation source 16 is moved to each of radiation source positions S1, S2, ..., Sc, ..., and Sn. The radiation source 16 is driven at each radiation source position to irradiate the breast M with radiation. The radiation detector 15 detects the radiation transmitted through the breast M to acquire projection images G1, G2, ..., Gc, ..., and Gn corresponding to the radiation source positions S1 to Sn, respectively. Here, the radiation source position Sc illustrated in Fig. 5 is a radiation source position where an optical axis X0 of the radiation emitted from the radiation source 16 is orthogonal to the detection surface 15A of the radiation detector 15. Hereinafter, in some cases, the radiation source position Sc is referred to as a reference radiation source position Sc. At each of the radiation source positions S1 to Sn, the same dose of radiation is emitted to the breast M. The plurality of acquired projection images Gi are stored in the storage 23.

The reconstruction unit 32 reconstructs the projection images Gi to generate the tomographic images in which the desired tomographic planes of the breast M have been highlighted. Specifically, the reconstruction unit 32 reconstructs the plurality of projection images Gi using a known back projection method, such as a simple back projection method or a filtered back projection method, to generate a plurality of tomographic images Dj (j = 1 to m) in each of the plurality of tomographic planes of the breast M as illustrated in Fig. 6. In this case, a three-dimensional coordinate position in a three-dimensional space including the breast M is set, pixel values at corresponding pixel positions in the plurality of projection images Gi are reconstructed for the set three-dimensional coordinate position, and pixel values at the coordinate positions are calculated. A three-dimensional image of the breast M is configured by the plurality of tomographic images Dj generated by the reconstruction.

The combination unit 33 generates a composite two-dimensional image CG0 using the plurality of tomographic images Dj. The composite two-dimensional image CG0 is a pseudo two-dimensional image corresponding to a simple two-dimensional image that is captured by irradiating the breast M with radiation emitted at the reference radiation source position Sc. In this embodiment, the combination unit 33 generates the composite two-dimensional image CG0 using an addition method. The addition method is a method that weights and adds the values of the corresponding pixels in each of the plurality of tomographic images Dj along a viewing direction from the reference radiation source position Sc to the radiation detector 15, that is, the optical axis X0 shown in Fig. 5 in a state in which the tomographic images Dj are stacked. In the addition method, a weight for each pixel during the weighting and addition is set to 11m in a case in which m is the number of tomographic images Dj. A method for generating the composite two-dimensional image CG0 is not limited to the addition method and a known technique, such as an averaging method, a minimum intensity projection method, or a maximum intensity projection method, can be applied.

The image set including the plurality of tomographic images Dj and the composite two-dimensional image CG0 generated as described above is transmitted to the PACS 7 through the network 5 by the communication unit 24 in response to a command from the input unit 4. In this case, the image set includes identification information (for example, an image ID, a patient name, and an imaging date and time) for uniquely identifying the image set. The image set transmitted to the PACS 7 is stored in the PACS 7. The image set may include at least one of the plurality of projection images Gi.

The image interpretation terminal 8 is a computer that is used by a radiologist who interprets a radiographic image to interpret a radiographic image and to make an interpretation report. The image interpretation terminal 8 includes an image display device according to an embodiment of the present disclosure. Therefore, an image display program according to this embodiment is installed in the image interpretation terminal 8. The image display program is stored in a storage device of a server computer connected to the network or a network storage in a state in which it can be accessed from the outside and is downloaded and installed in the computer as required. Alternatively, the program is recorded on a recording medium, such as a DVD or a CD-ROM, is distributed, and is installed in the computer from the recording medium.

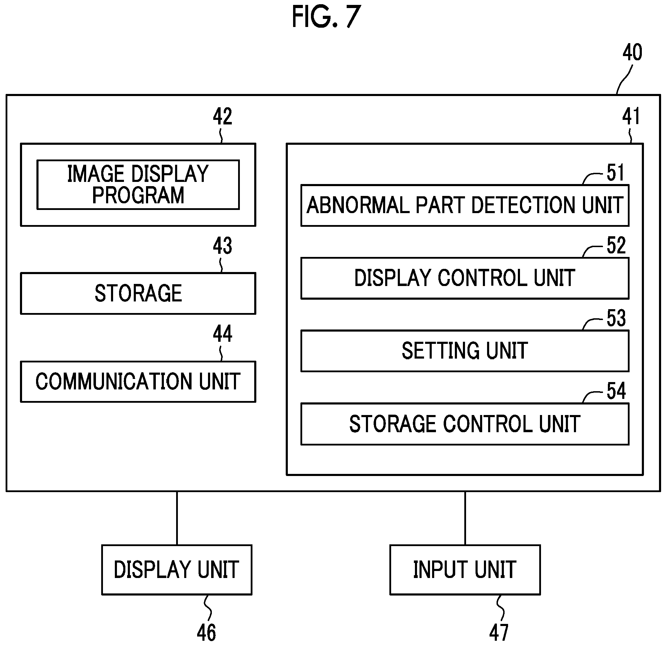

Fig. 7 is a diagram schematically illustrating a configuration of the image display device implemented by installing the image display program in the computer. As illustrated in Fig. 7, the image display device 40 comprises a CPU 41, a memory 42, a storage 43, and a communication unit 44 as a standard computer configuration. Further, the image display device 40 is connected to a display unit 46, such as a high-definition liquid crystal display for interpreting a radiographic image, and an input unit 47, such as a keyboard or a mouse.

The storage 43 consists of a storage device, such as a hard disk drive or an SSD, and stores various kinds of information including the image display program according to this embodiment. The storage 43 corresponds to a storage unit.

The memory 42 temporarily stores, for example, the image display program stored in the storage 43 in order to cause the CPU 41 to perform various processes. The image display program defines the following processes as the processes to be performed by the CPU 41: an abnormal part detection process that detects an abnormal part, such as a lesion, from the plurality of tomographic images Dj and the composite two-dimensional image CG0 included in the image set acquired from the PACS7; a display control process that displays, on the display unit 46, a tomographic image for which a display command is given among the plurality of tomographic images included in the image set; a setting process that sets the displayed tomographic image as having been displayed; and a storage control process that stores only the tomographic image set as having been displayed in the storage 43.

In a case in which the CPU 41 performs these processes according to the image display program such that the CPU 41 functions as an abnormal part detection unit 51, a display control unit 52, a setting unit 53, and a storage control unit 54.

The communication unit 44 is a network interface that controls the transmission of various kinds of information through the network 5. In a case in which the identification information of the acquired image set is input from the input unit 47, the communication unit 44 transmits the input identification information to the PACS 7 through the network 5. The PACS 7 transmits an image set corresponding to the received identification information to the image interpretation terminal 8 through the network 5. Then, the communication unit 44 receives the image set and stores the image set in the storage 43. The communication unit 44 transmits the setting information generated by the setting unit 53 as described below to the PACS 7 through the network 5.

The abnormal part detection unit 51 detects a lesion, such as calcification, as an abnormal part from the plurality of tomographic images Dj and the composite two-dimensional image CG0 included in the image set received by the communication unit 44. In this embodiment, the abnormal part is detected from the plurality of tomographic images Dj and the composite two-dimensional image CG0 by a known computer-aided diagnosis (CAD) algorithm. For example, a method described in JP2002-099896A can be used as the CAD algorithm. The method described in JP2002-099896A is a method that detects a calcified region using a shape filter corresponding to a calcified shadow.

The abnormal part detection unit 51 may comprise a discriminator that has been trained to detect an abnormal part by, for example, deep learning and detect the abnormal part from the plurality of tomographic images Dj and the composite two-dimensional image CG0 using the discriminator. Here, the composite two-dimensional image CG0 includes all of the abnormal parts included in the plurality of tomographic images Dj. Therefore, the abnormal part detection unit 51 associates the abnormal part detected in the plurality of tomographic images Dj with the abnormal part included in the composite two-dimensional image CG0. Specifically, the abnormal part included in the composite two-dimensional image CG0 is associated with the tomographic image Dj including the abnormal part.

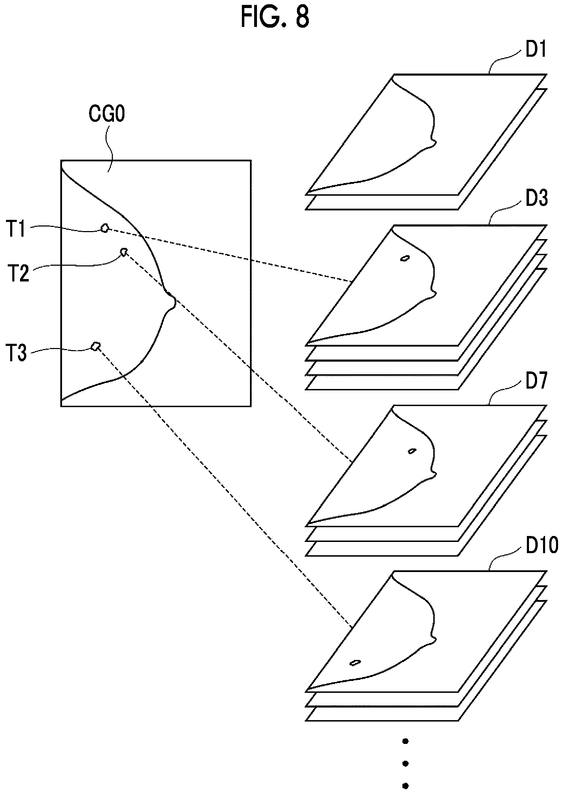

Fig. 8 is a diagram illustrating the association between an abnormal part and a tomographic image. As illustrated in Fig. 8, it is assumed that lesions T1, T2, and T3 are detected as the abnormal parts in the tomographic images D3, D7, and D10 among the plurality of tomographic images Dj, respectively, and the three lesions T1 to T3 are included in the composite two-dimensional image CG0. The abnormal part detection unit 51 aligns the tomographic images D3, D7, and D10 including the abnormal parts with the composite two-dimensional image CG0 to specify abnormal parts in the composite two-dimensional image CG0 which correspond to the abnormal parts included in the tomographic images D3, D7, and D10. In this embodiment, the tomographic image D3 is associated with the lesion T1, the tomographic image D7 is associated with the lesion T2, and the tomographic image D10 is associated with the lesion T3. Specifically, links to the tomographic images D3, D7, and D10 are generated in the regions of interest with a predetermined size including the lesions T1 to T3 in the composite two-dimensional image CG0.

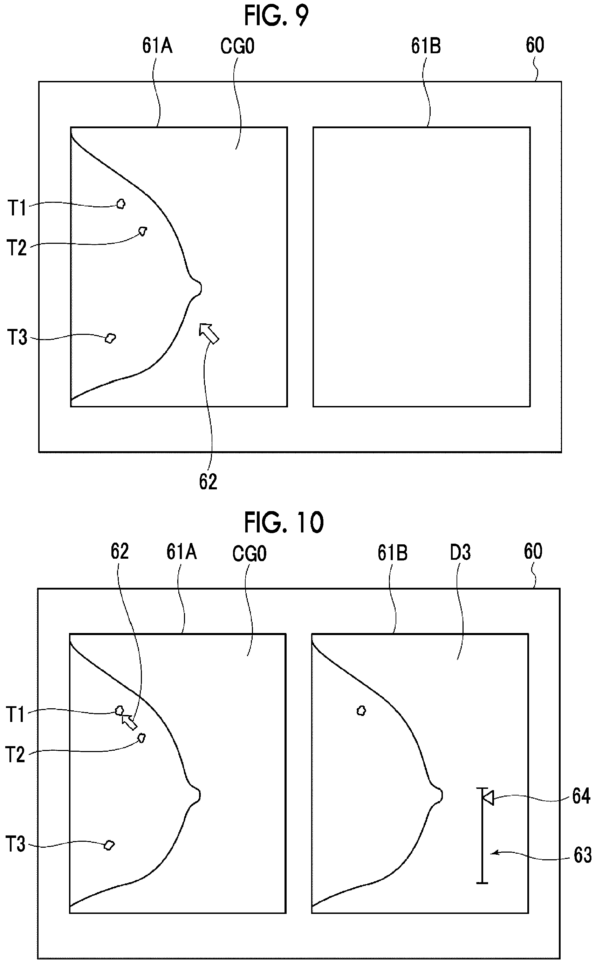

The display control unit 52 displays, on the display unit 46, a tomographic image for which a display command is given among the plurality of tomographic images Dj. Fig. 9 is a diagram illustrating a tomographic image display screen. As illustrated in Fig. 9, a display screen 60 includes a display region 61A for displaying the composite two-dimensional image CG0 and a display region 61B for displaying a tomographic image. Then, in a case in which a command to start image interpretation is input to the image interpretation terminal 8 by the radiologist who is an operator, the display control unit 52 displays the composite two-dimensional image CG0 in the display region 61A first, as illustrated in Fig. 9. The composite two-dimensional image CG0 includes the three lesions T1 to T3 detected by the abnormal part detection unit 51. In this state, no tomographic image is displayed in the display region 61B. In addition, a cursor 62 that is moved in response to a command from the input unit 47 is displayed on the display screen 60.

In a case in which the radiologist moves the cursor 62 with the input unit 47 and selects the lesion T1 in the composite two-dimensional image CG0, the display control unit 52 displays the tomographic image D3 including the lesion T1 in the display region 61B as illustrated in Fig. 10. Therefore, the radiologist can check the lesion T1 in the tomographic image D3 in detail. A scale 63 indicating the position of the displayed tomographic plane is displayed in the tomographic image D3 displayed in the display region 61B. In the scale 63, the position of the tomographic plane is indicated by the position on the scale indicated by a triangular mark 64. Further, in the scale 63, the upper side indicates the side of the breast M which comes into contact with the compression plate 17.

In contrast, in a case in which the radiologist selects the lesion T2 with the cursor 62, the display control unit 52 displays the tomographic image D7 including the lesion T2 in the display region 61B. In a case in which the radiologist selects the lesion T3, the tomographic image D10 including the lesion T3 is displayed in the display region 61B. In this embodiment, the tomographic image displayed in the display region 61B can be changed by a scroll button of the mouse forming the input unit 47. Therefore, the radiologist can check the tomographic images in the tomographic planes before and after the tomographic plane in which a lesion has been detected.

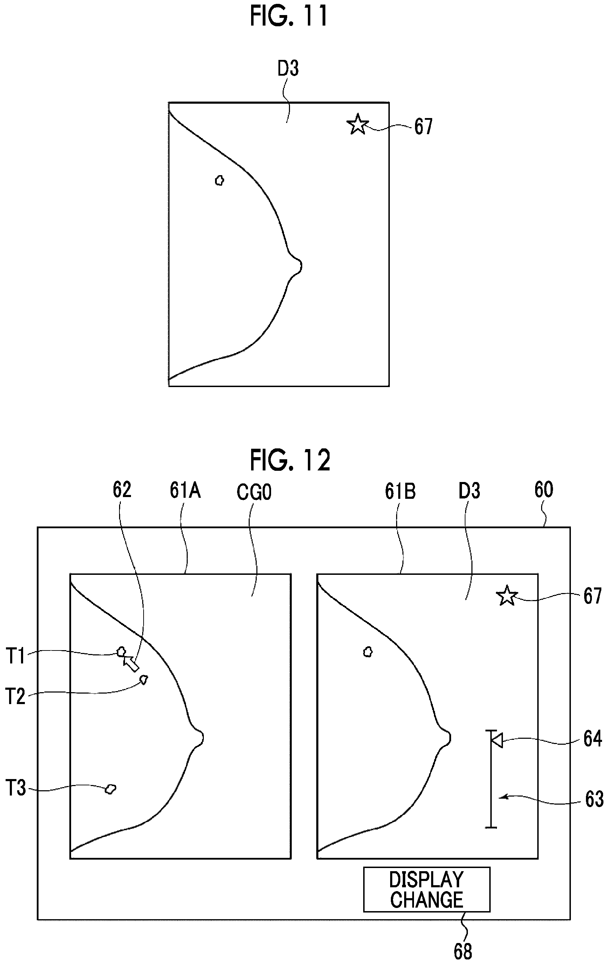

The setting unit 53 sets the displayed tomographic image among the plurality of tomographic images Dj as having been displayed. In this embodiment, the setting unit 53 sets a flag indicating that an image has been displayed in the header of the displayed tomographic image to set the tomographic image as having been displayed. In this embodiment, assuming that the tomographic images D3, D7, and D10 including the lesions T1 to T3 among the plurality of tomographic images Dj have been displayed, the setting unit 53 sets the tomographic images D3, D7, and D10 as having been displayed. In addition, the setting unit 53 may add a mark indicating that an image has been displayed to the displayed tomographic image. Fig. 11 is a diagram illustrating a tomographic image to which a mark indicating that an image has been displayed is added. As illustrated in Fig. 11, an asterisk mark 67 is added to the displayed tomographic image D3.

Further, the setting unit 53 may change the tomographic image set as having been displayed to a non-display state. For example, as illustrated in Fig. 12, a display change button 68 is displayed below the display region 61B. In a case in which the display change button 68 is selected using the input unit 47 during the display of the tomographic image D3, the setting of the displayed tomographic image D3 may be changed from a "displayed state" to a "non-display state". In a case in which the mark 67 has been added to the tomographic image D3 as illustrated in Fig. 12 and the display change button 68 is selected, the setting unit 53 deletes the mark 67 from the tomographic image D3. In a case in which the display change button 68 is selected again, the setting unit 53 may change the setting from the "non-display state" to the "displayed state". In this case, the setting unit 53 may display the mark 67 again.

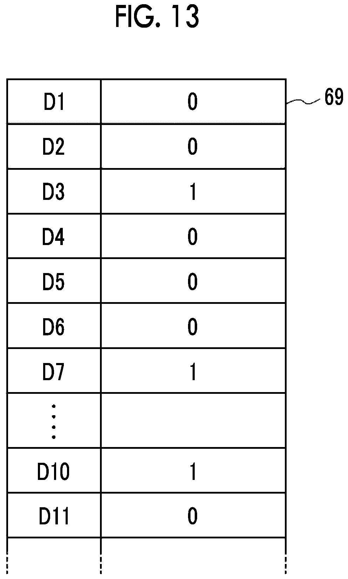

In a case in which the radiologist completes image interpretation, the radiologist inputs an end command to the image display device 40 through the input unit 47. The setting unit 53 generates setting information for specifying the tomographic image set as having been displayed in response to the end command. Fig. 13 illustrates the setting information. As illustrated in Fig. 13, in setting information 69, a flag indicating whether or not an image has been displayed is set to each of the plurality of tomographic images Dj. That is, as illustrated in Fig. 13, in the setting information 69, a flag "1" indicating that an image has been displayed is set to the displayed tomographic images D3, D7, and D10 and a flag "0" is set to the tomographic images which have not been displayed. In addition, the setting information 69 includes the identification information of an image set including the tomographic images whose setting information 69 has been set. Further, the setting information includes abnormal part information indicating, for example, the position and size of an abnormal part such as a lesion detected by the abnormal part detection unit 51.

In a case in which the setting information 69 is generated, the communication unit 44 transmits the setting information 69 to the PACS 7 through the network 5.

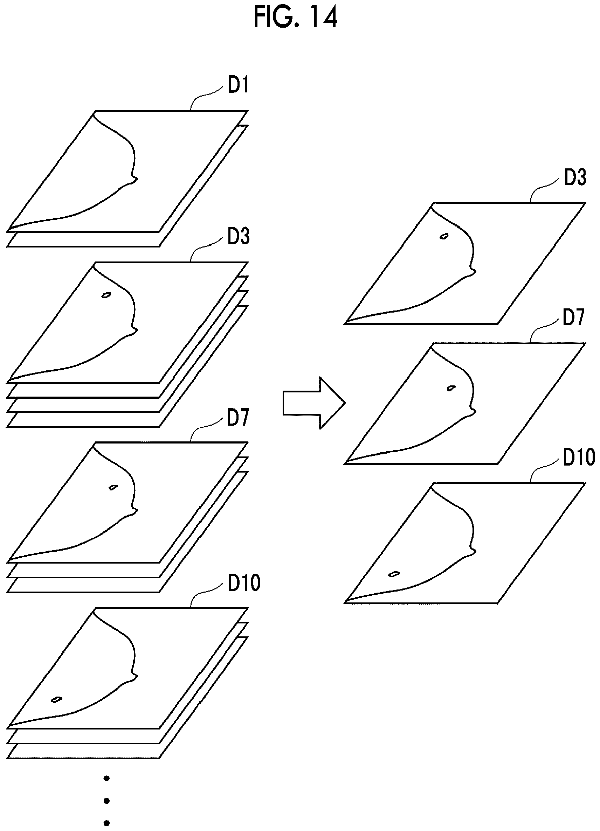

The storage control unit 54 deletes tomographic images other than the tomographic image set as having been displayed among the plurality of tomographic images Dj included in the image set stored in the storage 43. Fig. 14 is a diagram illustrating the deletion of the tomographic images. As illustrated in Fig. 14, the storage control unit 54 deletes tomographic images other than the tomographic images D3, D7, and D10 set as having been displayed among the plurality of tomographic images Dj stored in the storage 43. As a result, for the interpreted image set, only the displayed tomographic images D3, D7, and D10 and the composite two-dimensional image CG0 are stored in the storage 43.

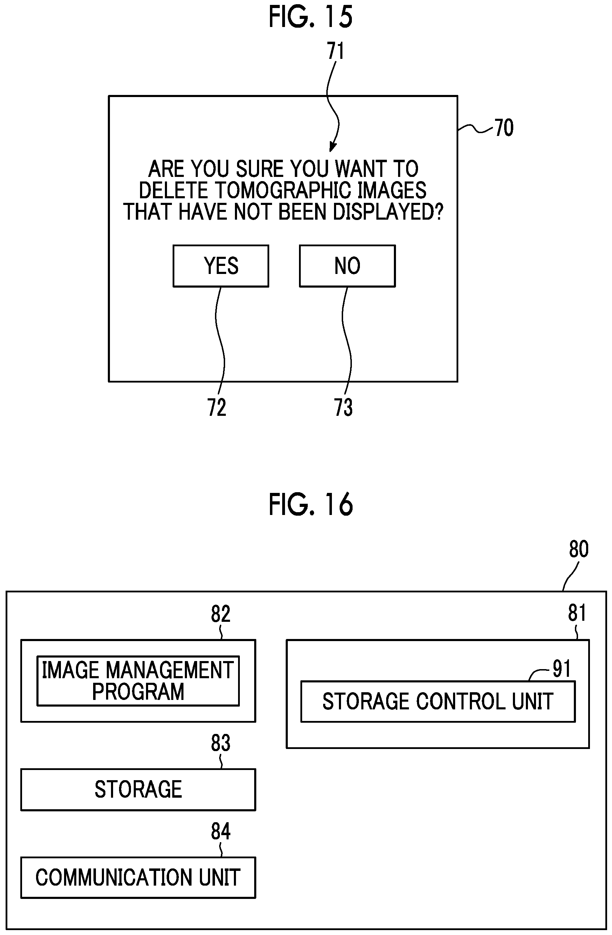

In a case in which the storage control unit 54 deletes tomographic images other than the tomographic images D3, D7, and D10 set as having been displayed, a deletion confirmation screen may be displayed on the display unit 46. Fig. 15 is a diagram illustrating a screen for confirming the deletion of other tomographic images. As illustrated in Fig. 15, a text 71 of "Are you sure you want to delete tomographic images that have not been displayed?", a YES button 72, and a NO button 73 are displayed on a confirmation screen 70. In a case in which the radiologist wants to delete the tomographic images, the radiologist selects the YES button 72 using the input unit 47. Then, the storage control unit 54 deletes tomographic images other than the tomographic images D3, D7, and D10 set as having been displayed among the plurality of tomographic images Dj stored in the storage 43. In contrast, in a case in which the radiologist selects the NO button 73, the storage control unit 54 does not perform any process. As a result, the state in which all of the plurality of tomographic images Dj included in the interpreted image set are stored in the storage 43 is maintained.

Further, instead of deleting the tomographic images other than the tomographic images D3, D7, and D10 set as having been displayed, the storage control unit 54 sets the compression rate of the tomographic images other than the tomographic images D3, D7, and D10 set as having been displayed to be higher than the compression rate of the tomographic images D3, D7, and D10 set as having been displayed. For example, the compression rate of the displayed tomographic images D3, D7, and D10 may not be changed and the compression rate of the other tomographic images may be set to 50% of the compression rate in a case in which the other tomographic images have been stored. Only in a case in which a confirmation screen that asks the radiologist whether or not to increase the compression rate of the tomographic images other than the tomographic images D3, D7, and D10 set as having been displayed and to store the tomographic images is displayed and the radiologist inputs a command to increase the compression rate of the tomographic images and to store the tomographic images, the storage control unit 54 may increase the compression rate of the tomographic images other than the tomographic images D3, D7, and D10 set as having been displayed and store the tomographic images in the storage 43.

The PACS 7 is a server computer for storing and managing the images transmitted from the radiography system 1. The PACS 7 includes an image management device according to the embodiment of the present disclosure. Therefore, an image management program according to this embodiment is installed in the PACS 7. Hereinafter, only the processes performed by the image management device according to the present disclosure in the PACS 7 will be described and the description of the processes performed by the PACS 7 other than the processes performed by the image management device will be omitted.

Fig. 16 is a diagram schematically illustrating a configuration of the image management device implemented by installing the image management program in a computer. As illustrated in Fig. 16, an image management device 80 comprises a CPU 81, a memory 82, a storage 83, and a communication unit 84 as a standard computer configuration.

The storage 83 consists of a storage device, such as a large-capacity hard disk drive or SSD, and stores various kinds of information including the image management program according to this embodiment in addition to, for example, the received tomographic images. The storage 83 corresponds to a storage unit.

The memory 82 temporarily stores, for example, the image management program stored in the storage 83 in order to cause the CPU 81 to perform various processes. The image management program defines, as the process to be performed by the CPU 81, a storage control process that stores the image set acquired from the radiography system 1 in the storage 83 and deletes tomographic images other than the tomographic image set as having been displayed from the storage 83 on the basis of the setting information 69 transmitted from the image interpretation terminal 8.

Then, the CPU 81 performs the storage control process according to the image management program such that the CPU 81 functions as a storage control unit 91.

Here, the communication unit 84 is a network interface that controls the transmission of various kinds of information through the network 5. The communication unit 84 receives the image set transmitted from the radiography system 1 through the network 5. The image set corresponding to the identification information transmitted from the image interpretation terminal 8 is transmitted to the image interpretation terminal 8 through the network 5. In addition, the communication unit 84 receives the setting information 69 transmitted from the image interpretation terminal 8.

The storage control unit 91 stores the image set received by the communication unit 84 from the radiography system 1 in the storage 83 so as to be associated with the identification information. Further, the storage control unit 91 specifies an image set corresponding to the identification information included in the setting information 69 received from the image interpretation terminal 8 by the communication unit 84 on the basis of the setting information 69 and deletes tomographic images other than the tomographic images set as having been displayed in the image set from the storage 83. For example, in a case in which the tomographic images D3, D7, and D 10 among the plurality of tomographic images Dj included in the specified image set are set as having been displayed, the storage control unit 91 deletes tomographic images other than the tomographic images D3, D7, and D10 set as having been displayed among the plurality of tomographic images Dj included in the image set stored in the storage 83 as in the case described with reference to Fig. 14. Therefore, only the displayed tomographic images D3, D7, and D10 and the composite two-dimensional image CG0 in the interpreted image set are stored in the storage 83.

Further, instead of deleting tomographic images other than the tomographic images D3, D7, and D10 set as having been displayed, the storage control unit 91 may set the compression rate of the tomographic images other than the tomographic images D3, D7, and D10 set as having been displayed to be higher than the compression rate of the tomographic images D3, D7, and D10 set as having been displayed and may store the other tomographic images. For example, the displayed tomographic images D3, D7, and D10 may be maintained at the stored compression rate and the compression rate of the other tomographic images may be set to 50% of the compression rate in a case in which the other tomographic images have been stored.

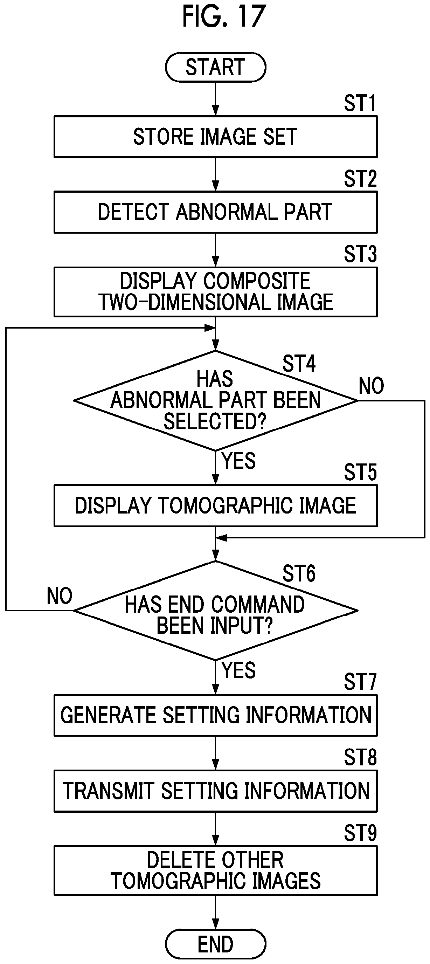

Next, a process performed in this embodiment will be described. It is assumed that the plurality of tomographic images Dj and the composite two-dimensional image CG0 acquired by the radiography system 1 are stored in the PACS 7 so as to be associated with identification information. First, a process performed by the image display device 40 included in the image interpretation terminal 8 will be described. Fig. 17 is a flowchart illustrating the process performed in the image display device 40. First, the image set desired to be interpreted which has been transmitted from the PACS 7 is received by the communication unit 24 through the network 5 and is stored in the storage 43 (Step ST1). Then, the abnormal part detection unit 51 detects an abnormal part from the plurality of tomographic images Dj and the composite two-dimensional image CG0 included in the image set (Step ST2).

Then, the display control unit 52 displays the composite two-dimensional image CG0 on the display unit 46 (Step ST3). The display control unit 52 determines whether or not a lesion, that is, an abnormal part included in the composite two-dimensional image CG0 has been selected by a command from the input unit 47 (Step ST4). In a case in which the determination result in Step ST4 is "No", the process proceeds to Step ST6. In a case in which the determination result in Step ST4 is "Yes", a tomographic image corresponding to the designated abnormal part is displayed on the display unit 46 (Step ST5). Then, the display control unit 52 determines whether or not an end command has been input from the input unit 47 (Step ST6). In a case in which the determination result in Step ST6 is "No", the process returns to Step ST4 and Steps ST4 to ST6 are repeatedly performed.

In a case in which the determination result in Step ST6 is "Yes", the setting unit 53 sets the tomographic image displayed on the display unit 46 as having been displayed and generates the setting information 69 (Step ST7). Then, the setting unit 53 transmits the setting information 69 to the PACS 7 through the network 5 using the communication unit 44 (Step ST8). Further, the storage control unit 54 deletes tomographic images other than the tomographic image set as having been displayed among the plurality of tomographic images Dj included in the interpreted image set stored in the storage 43 (Step ST9). Then, the process ends.

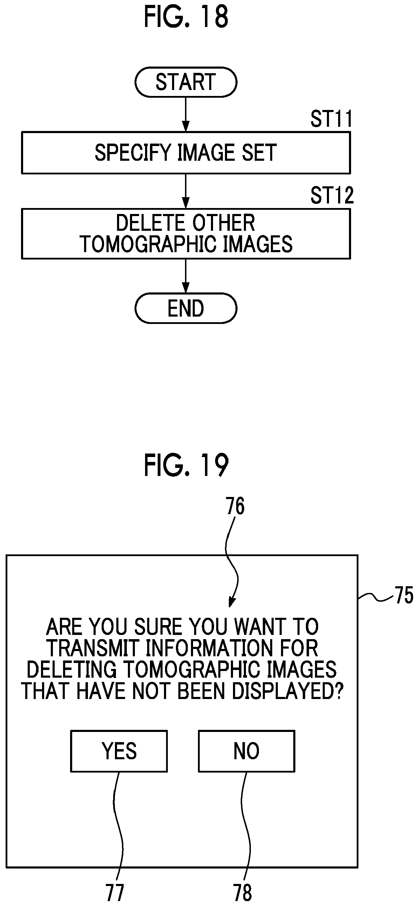

Fig. 18 is a flowchart illustrating a process performed in the image management device 80. The process is started by the reception of the setting information 69 transmitted from the image interpretation terminal 8 by the communication unit 84 and the storage control unit 91 specifies a image set with reference to the identification information of the image set included in the setting information 69 (Step ST11). Then, the storage control unit 91 deletes tomographic images other than the tomographic image set as having been displayed among the plurality of tomographic images Dj included in the specified image set (Step ST12). Then, the process ends.

As described above, in this embodiment, among the plurality of tomographic images Dj acquired by tomosynthesis imaging, the tomographic image for which a display command is given is displayed on the display unit 46 in the image interpretation terminal 8 and the displayed tomographic image is set as having been displayed. Therefore, the image interpretation terminal 8 can delete tomographic images other than the tomographic image set as having been displayed or can increase the compression rate of the other tomographic images. Further, the PACS 7 can delete tomographic images other than the tomographic image set as having been displayed or can increase the compression rate of the other tomographic images, on the basis of the received setting information 69. Therefore, according to this embodiment, it is possible to appropriately reduce the capacity of the tomographic images acquired by tomosynthesis imaging.

In the above-described embodiment, before the setting information 69 is transmitted from the image display device 40 to the PACS 7, a confirmation screen for allowing the radiologist to confirm whether or not to transmit the setting information 69 to the PACS 7 such that the PACS 7 deletes the tomographic image that has not been displayed may be displayed on the display unit 46. Fig. 19 is a diagram illustrating a screen for confirming whether or not to permit the transmission of the setting information. As illustrated in Fig. 19, a text 76 of "Are you sure you want to transmit information for deleting tomographic images that have not been displayed?", a YES button 77, and a NO button 78 are displayed on the confirmation screen 75. In a case in which the radiologist wants to delete the tomographic images that have not been displayed in the PACS 7, the radiologist selects the YES button 77 using the input unit 47. Then, the communication unit 44 transmits the setting information 69 to the PACS 7 through the network 5. Then, the PACS 7 deletes tomographic images other than the tomographic image set as having been displayed from the storage 83 on the basis of the setting information 69. In contrast, in a case in which the operator selects the NO button 78, the communication unit 44 does not perform any process. As a result, the interpreted image set is stored in the PACS 7 without any change.

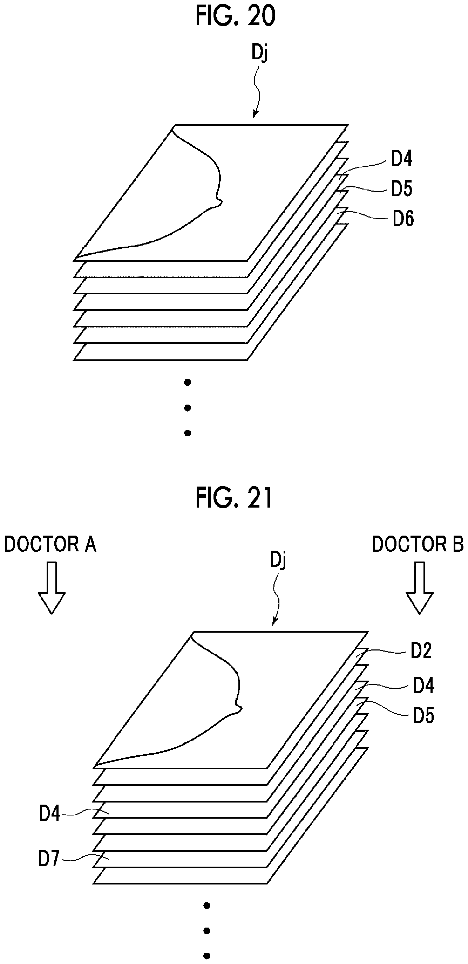

In the above-described embodiment, the image display device 40 of the image interpretation terminal 8 sets the tomographic image displayed on the display unit 46 as having been displayed. However, the present disclosure is not limited thereto. One or more tomographic images in the tomographic planes adjacent to the displayed tomographic image may be set as having been displayed. For example, as illustrated in Fig. 20, in a case in which a tomographic image D5 is displayed among the plurality of tomographic images Dj, the setting unit 53 may set tomographic images D4 and D6 in the tomographic planes which are vertically adjacent to the tomographic image D5 as having been displayed. In addition, the number of adjacent tomographic images on the upper side or the lower side is not limited to one and may be equal to or greater than two. Further, only the tomographic images in one or more tomographic planes adjacent on the upper side of the tomographic plane of the displayed tomographic image or only the tomographic images in one or more tomographic planes adjacent on the lower side of the tomographic plane of the displayed tomographic image may be set as having been displayed.

In the above-described embodiment, in some cases, one image set may be interpreted by a plurality of radiologists. In this case, the image set is transmitted to the image interpretation terminals 8 of the plurality of different radiologists and is interpreted. In image interpretation, in some cases, the tomographic image to be interpreted may vary depending on the radiologist. For example, as illustrated in Fig. 21, it is assumed that, among the plurality of tomographic images Dj, a doctor A displays the tomographic images D4 and D7 and a doctor B displays the tomographic images D2, D4 and D5. In this case, setting information 69 indicating that the tomographic images D4 and D7 have been set as having been displayed is transmitted from the image interpretation terminal 8 of the doctor A to the PACS 7. In contrast, setting information 69 in which the tomographic images D2, D4, and D5 have been set as having been displayed is transmitted to the image interpretation terminal 8 of the doctor B to the PACS 7B.

The storage control unit 91 of the image management device 80 in the PACS 7 deletes tomographic images other than the tomographic images set as having been displayed among the plurality of tomographic images Dj stored in the storage 83 on the basis of the received setting information 69. In this case, the storage control unit 91 deletes tomographic images other than all of the tomographic images set as having been displayed which are included in the setting information 69 transmitted from the image interpretation terminal 8 of the doctor A and the setting information 69 transmitted from the image interpretation terminal 8 of the doctor B. That is, the storage control unit 91 deletes tomographic images other than the tomographic images D2, D4, D5, and D7 set as having been displayed in the image interpretation terminals 8 of both the doctor A and the doctor B among the plurality of tomographic images Dj from the storage 83.

In addition, the storage control unit 91 may delete tomographic images other than the common tomographic image set as having been displayed which is included in the setting information 69 transmitted from the image interpretation terminal 8 of the doctor A and the setting information 69 transmitted from the image interpretation terminal 8 of the doctor B. In this case, the tomographic image displayed on both the image interpretation terminal 8 of the doctor A and the image interpretation terminal 8 of the doctor B is the tomographic image D4. Therefore, the storage control unit 91 deletes tomographic images other than the tomographic image D4 among the plurality of tomographic images Dj from the storage 83.

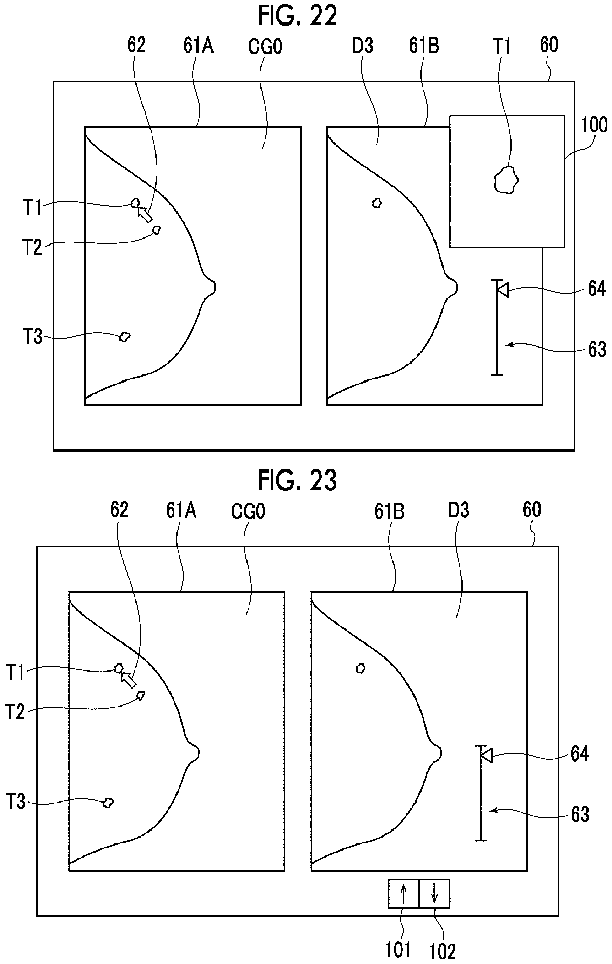

Further, in the above-described embodiment, the image display device 40 may display an enlarged image obtained by enlarging a region of interest that includes an abnormal part included in the displayed tomographic image, in addition to the tomographic image displayed in the display region 61B of the display screen 60. Fig. 22 is a diagram illustrating a state in which the enlarged image is displayed. As illustrated in Fig. 22, in a case in which the radiologist selects the lesion T1 in the composite two-dimensional image CG0, the tomographic image D3 including the lesion T1 is displayed in the display region 61B and an enlarged image 100 of the region of interest including the lesion T1 is displayed so as to be partially superimposed on the tomographic image D3. The radiologist can easily check the state of the lesion T1 included in the displayed tomographic image D3 with reference to the enlarged image 100.

Further, in the above-described embodiment, the tomographic image displayed on the display screen 60 may be switched and displayed. For example, as illustrated in Fig. 23, switching buttons 101 and 102 for switching the tomographic image displayed in the display region 61B may be displayed below the display region 61B of the display screen 60. In this case, the switching buttons 101 and 102 may be selected such that the tomographic image without including an abnormal part detected by the abnormal part detection unit 51 is not displayed and only the tomographic image including an abnormal part is displayed. For example, in a case in which a lesion is included in the tomographic images D3, D7, and D10 among the plurality of tomographic images Dj, only the tomographic images D3, D7, and D10 may be displayed in the display region 61B so as to be switched in response to a command for the switching buttons 101 and 102.

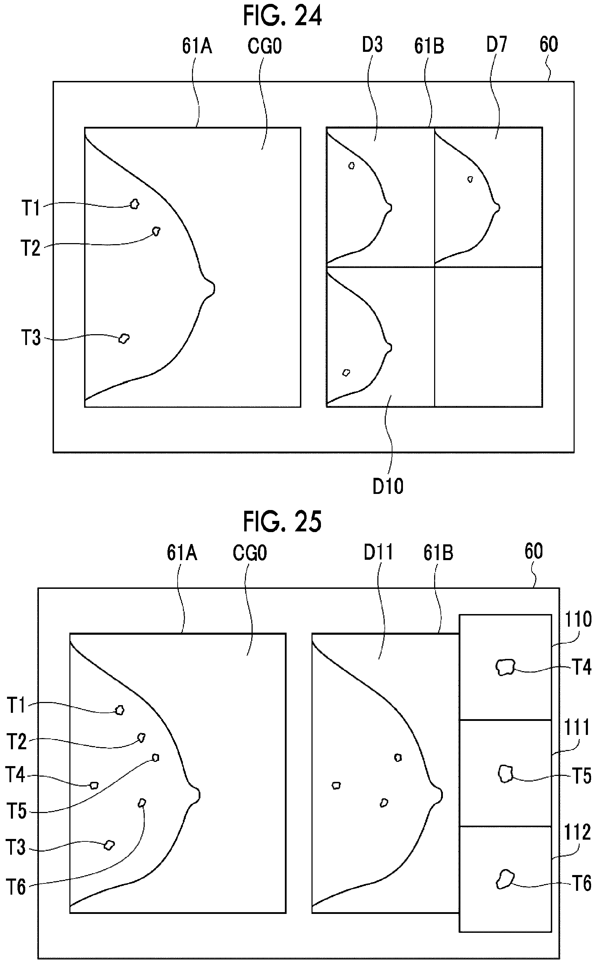

In the above-described embodiment, the tomographic images in which the abnormal part has been detected by the abnormal part detection unit 51 may be displayed side by side in the display region 61B of the display screen 60. In a case in which the tomographic images D3, D7, and D10 among the plurality of tomographic images Dj include lesions, the tomographic images D3, D7, and D10 may be displayed side by side in the display region 61B as illustrated in Fig. 24. In a case in which any one of the tomographic images D3, D7, and D10 displayed side by side is selected, the selected tomographic image may be enlarged and displayed in the display region 61B as illustrated in Fig. 10. In this case, the region of interest including a lesion in the enlarged tomographic image may be enlarged and displayed as illustrated in Fig. 22.

In the above-described embodiment, a plurality of abnormal parts are likely to be included in one tomographic image. In this case, in addition to the tomographic image displayed in the display region 61B of the display screen 60, the image of the region of interest including each of the plurality of abnormal parts may be displayed. Fig. 25 is a diagram illustrating a state in which a plurality of regions of interest are displayed. As illustrated in Fig. 25, in a case in which a plurality of lesions T4 to T6 are included in the tomographic image D11 displayed in the display region 61B and the radiologist selects the lesion T4 in the composite two-dimensional image CG0, the tomographic image D11 including the lesion T4 is displayed in the display region 61B and the enlarged images 110 to 112 of the regions of interest including the lesions T4 to T6 are displayed so as to be partially superimposed on the tomographic image D 11. The radiologist can easily check the states of the lesions T4 to T6 included in the displayed tomographic image D 11 with reference to the enlarged images 110 to 112.

In the above-described embodiment, the received image set is stored in the image interpretation terminal 8. However, the received image set may not be stored in the storage 43 in the image interpretation terminal 8.

Further, in the above-described embodiment, in a case in which tomosynthesis imaging is performed for the breast M, the images of each of the left and right breasts M may be captured in a cranio-caudal (CC) direction (referred to as a CC direction) and a medio-lateral oblique (MLO) direction (referred to as an MLO direction). In this case, the composite two-dimensional image CG0 and the tomographic images acquired only by capturing the images of one of the left and right breasts M in the CC direction may be interpreted and the composite two-dimensional image CG0 and the tomographic images acquired by capturing the images in the MLO direction may not be interpreted. In this case, all of the tomographic images acquired by imaging in the CC direction may be set as having been displayed. In this case, in the setting information 69 transmitted from the image display device 40 of the image interpretation terminal 8 to the PACS 7, "1" which is a flag indicating that an image has been displayed is set to all of the tomographic images captured in the CC direction and "0" which is a flag indicating that an image has not been displayed is set to all of the tomographic images captured in the MLO direction.

Therefore, in a case in which the image set is stored in the storage 43 of the image interpretation terminal 8, all of the tomographic images in the MLO direction are deleted and all of the tomographic images in the CC direction are stored. In the PACS 7, all of the tomographic images in the MLO direction are deleted and all of the tomographic images in the CC direction are stored. Therefore, it is possible to reduce the capacity of the image set.

Further, in the above-described embodiment, in the image display device 40 of the image interpretation terminal 8, the abnormal part detection unit 51 detects an abnormal part from the tomographic images Dj using CAD. However, the present disclosure is not limited thereto. In addition, the following configuration may be used: the tomographic images Dj are displayed on the display unit 46 and the radiologist selects an abnormal part included in the tomographic images Dj using the input unit 47.

Further, the radiation in the above-described embodiment is not particularly limited. For example, α-rays or γ-rays can be applied in addition to the X-rays.

In the above-described embodiment, for example, the following various processors can be used as the hardware structure of processing units performing various processes, such as the abnormal part detection unit 51, the display control unit 52, the setting unit 53, and the storage control unit 54 of the image display device 40 and the storage control unit 91 of the image management device 80. The various processors include, for example, a CPU which is a general-purpose processor executing software (program) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or a combination of two or more processors of the same type or different types (for example, a combination of a plurality of FPGAs or a combination of a CPU and an FPGA). In addition, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As such, various processing units are configured by using one or more of the various processors as a hardware structure.

In addition, specifically, an electric circuit (circuitry) obtained by combining circuit elements, such as semiconductor elements, can be used as the hardware structure of the various processors.

## Claims

1. An image display device comprising:
a display control unit (52) that displays, on a display unit (46), a tomographic image for which a display command is given among a plurality of tomographic images acquired by performing tomosynthesis imaging for an object;
a setting unit (53) that sets the displayed tomographic image as having been displayed; and
a storage control (54) unit that stores the tomographic image set as having been displayed in a storage unit (43), and stores tomographic images other than the tomographic image set as having been displayed at a compression rate higher than a compression rate of the tomographic image set as having been displayed, or deletes tomographic images other than the tomographic image set as having been displayed.

2. The image display device according to claim 1, further comprising:
an abnormal part detection unit (51) that detects an abnormal part from the tomographic image,
wherein the display control unit (52) displays a region of interest including the abnormal part on the display unit (46) in a case in which a tomographic image in which the abnormal part has been detected by the abnormal part detection unit (51) is displayed.

3. The image display device according to claim 1,
wherein the display control unit (46) displays a composite two-dimensional image generated by combining the plurality of tomographic images on the display unit (46) and displays a tomographic image including an abnormal part designated in the composite two-dimensional image on the display unit (46).

4. The image display device according to claim 1,
wherein, in a case in which the tomosynthesis imaging is performed for the object in a plurality of directions and one of the plurality of tomographic images is displayed on the display unit (46) for any one of the plurality of imaging directions, the setting unit (53) sets all of the tomographic images in the imaging direction as having been displayed.

5. The image display device according to claim 1,
wherein the setting unit (53) further sets at least one tomographic image in a tomographic plane which is adjacent to the displayed tomographic image as having been displayed.

6. The image display device according to claim 1,
wherein the setting unit (53) changes the tomographic image set as having been displayed to a non-display state in response to a command from an operator.

7. The image display device according to claim 1,
wherein the object is a breast.

8. A system comprising :
the image display device according to claim 1; and
an image management device comprising:
a storage unit (43) that stores a plurality of tomographic images acquired by performing tomosynthesis imaging for an object; and
a storage control unit (54) that deletes tomographic images other than the tomographic image set as having been displayed from the storage unit (43) on the basis of the setting result of the setting unit (53) of the image display device.

9. The system according to claim 8,
wherein the storage control unit (54) deletes the other tomographic images from the storage unit (43) on the basis of the setting result of the setting unit (53) in at least one of a plurality of the image display devices.

10. A system comprising:
the image display device according to claim 1; and
an image management device comprising:
a storage unit (43) that stores a plurality of tomographic images acquired by performing tomosynthesis imaging for an object; and
a storage control unit (54) that sets a compression rate of tomographic images other than the tomographic image set as having been displayed to be higher than a compression rate of the tomographic image set as having been displayed on the basis of the setting result of the setting unit (53) of the image display device and stores the plurality of tomographic images in the storage unit (43).

11. The system according to claim 10,
wherein the storage control unit (54) sets the compression rate of the other tomographic images to be higher than the compression rate of the tomographic image set as having been displayed on the basis of the setting result of the setting unit (53) in at least one of a plurality of the image display devices and store the plurality of tomographic images in the storage (43).

12. An image display method comprising:
displaying, on a display unit (46), a tomographic image for which a display command is given among a plurality of tomographic images acquired by performing tomosynthesis imaging for an object;
setting the displayed tomographic image as having been displayed;
storing the tomographic image set as having been displayed in a storage unit (43); and
storing tomographic images other than the tomographic image set as having been displayed at a compression rate higher than a compression rate of the tomographic image set as having been displayed, or deleting tomographic images other than the tomographic image set as having been displayed.

13. An image management method comprising:
storing a plurality of tomographic images acquired by performing tomosynthesis imaging for an object in a storage unit (43) and
deleting tomographic images other than the tomographic image set as having been displayed from the storage unit (43) on the basis of the setting result of the image display device according to claim 1.

14. An image management method comprising:
storing a plurality of tomographic images acquired by performing tomosynthesis imaging for an object in a storage unit (43); and
setting a compression rate of tomographic images other than the tomographic image set as having been displayed to be higher than a compression rate of the tomographic image set as having been displayed on the basis of the setting result of the setting unit (53) in the image display device according to claim 1 and storing the plurality of tomographic images in the storage unit (43).

15. A non-transitory computer-readable storage medium that stores an image display program that causes a computer to perform:
displaying, on a display unit (46), a tomographic image for which a display command is given among a plurality of tomographic images acquired by performing tomosynthesis imaging for an object;
setting the displayed tomographic image as having been displayed;
storing the tomographic image set as having been displayed in a storage unit (43); and
storing tomographic images other than the tomographic image set as having been displayed at a compression rate higher than a compression rate of the tomographic image set as having been displayed, or delete tomographic images other than the tomographic image set as having been displayed.

16. A non-transitory computer-readable storage medium that stores an image management program that causes a computer to perform:
storing a plurality of tomographic images acquired by performing tomosynthesis imaging for an object in a storage unit (43); and
deleting tomographic images other than the tomographic image set as having been displayed from the storage unit (43) on the basis of the setting result of the setting unit (53) in the display device according to claim 1.

17. A non-transitory computer-readable storage medium that stores an image management program that causes a computer to perform:
storing a plurality of tomographic images acquired by performing tomosynthesis imaging for an object in a storage unit (43); and
setting a compression rate of tomographic images other than the tomographic image set as having been displayed to be higher than a compression rate of the tomographic image set as having been displayed on the basis of the setting result of the setting unit (53) in the image display device according to claim 1 and storing the plurality of tomographic images in the storage unit (43).

## Patentansprüche

1. Bildanzeigevorrichtung, umfassend:
eine Anzeigesteuereinheit (52), die auf einer Anzeigeeinheit (46) ein Tomographie-Bild, für das ein Anzeigebefehl gegeben wird, aus einer Vielzahl von Tomographie-Bildern anzeigt, die durch Durchführen von Tomosynthese-Bildgebung für ein Objekt erfasst wurden;
eine Einstelleinheit (53), die das angezeigte Tomographie-Bild als angezeigt einstellt; und
eine Speichersteuereinheit (54), die das Tomographie-Bild, das als angezeigt eingestellt ist, in einer Speichereinheit (43) speichert und andere Tomographie-Bilder als das Tomographie-Bild, das als angezeigt eingestellt ist, mit einer Kompressionsrate speichert, die höher ist als die Kompressionsrate des Tomographie-Bildes, das als angezeigt eingestellt ist, oder andere Tomographie-Bilder als das Tomographie-Bild, das als angezeigt eingestellt ist, löscht.

2. Bildanzeigevorrichtung nach Anspruch 1, weiter umfassend:
eine Einheit (51) zur Erkennung abnormaler Teile, die einen abnormalen Teil aus dem Tomographie-Bild erkennt,
wobei die Anzeigesteuereinheit (52) einen Bereich von Interesse, der den abnormalen Teil beinhaltet, auf der Anzeigeeinheit (46) in einem Fall anzeigt, in dem ein Tomographie-Bild angezeigt wird, in dem der abnormale Teil durch die Einheit (51) zur Erkennung abnormaler Teile erkannt wurde.

3. Bildanzeigevorrichtung nach Anspruch 1,
wobei die Anzeigesteuereinheit (46) ein zusammengesetztes zweidimensionales Bild, das durch Kombinieren der Vielzahl von Tomographie-Bildern erzeugt wird, auf der Anzeigeeinheit (46) anzeigt und ein Tomographie-Bild, das einen abnormalen Teil beinhaltet, der in dem zusammengesetzten zweidimensionalen Bild bezeichnet ist, auf der Anzeigeeinheit (46) anzeigt.

4. Bildanzeigevorrichtung nach Anspruch 1,
wobei in einem Fall, in dem die Tomosynthese-Bildgebung für das Objekt in einer Vielzahl von Richtungen durchgeführt wird und eines der Vielzahl von Tomographie-Bildern auf der Anzeigeeinheit (46) für eine der Vielzahl von Bildgebungsrichtungen angezeigt wird, die Einstellungseinheit (53) alle Tomographie-Bilder in der Bildgebungsrichtung als angezeigt einstellt.

5. Bildanzeigevorrichtung nach Anspruch 1,
wobei die Einstelleinheit (53) weiter mindestens ein Tomographie-Bild in einer Tomographie-Ebene, die an das angezeigte Tomographie-Bild angrenzt, als angezeigt einstellt.

6. Bildanzeigevorrichtung nach Anspruch 1,
wobei die Einstelleinheit (53) das Tomographie-Bild, das als angezeigt eingestellt ist, als Reaktion auf einen Befehl von einem Bediener in einen Nicht-Anzeigezustand ändert.

7. Bildanzeigevorrichtung nach Anspruch 1,
wobei es sich bei dem Objekt um eine Brust handelt.

8. System, umfassend:
die Bildanzeigevorrichtung nach Anspruch 1; und
eine Bildverwaltungsvorrichtung, umfassend:
eine Speichereinheit (43), die eine Vielzahl von Tomographie-Bildern speichert, die durch Durchführen von Tomosynthese-Bildgebung für ein Objekt erfasst wurden; und
eine Speichersteuereinheit (54), die auf Grundlage des Einstellungsergebnisses der Einstellungseinheit (53) der Bildanzeigevorrichtung andere Tomographie-Bilder als das Tomographie-Bild, das als angezeigt eingestellt ist, aus der Speichereinheit (43) löscht.

9. System nach Anspruch 8,
wobei die Speichersteuereinheit (54) die anderen Tomographie-Bilder aus der Speichereinheit (43) auf Grundlage des Einstellungsergebnisses der Einstellungseinheit (53) in mindestens einer einer Vielzahl der Bildanzeigevorrichtungen löscht.

10. System, umfassend:
die Bildanzeigevorrichtung nach Anspruch 1; und
eine Bildverwaltungsvorrichtung, umfassend:
eine Speichereinheit (43), die eine Vielzahl von Tomographie-Bildern speichert, die durch Durchführen von Tomosynthese-Bildgebung für ein Objekt erfasst wurden; und
eine Speichersteuereinheit (54), die auf Grundlage des Einstellungsergebnisses der Einstellungseinheit (53) der Bildanzeigevorrichtung eine Kompressionsrate anderer Tomographie-Bilder als des Tomographie-Bildes, das als angezeigt eingestellt ist, höher einstellt als eine Kompressionsrate des Tomographie-Bildes, das als angezeigt eingestellt ist, und die Vielzahl von Tomographie-Bildern in der Speichereinheit (43) speichert.

11. System nach Anspruch 10,
wobei die Speichersteuereinheit (54) auf Grundlage des Einstellungsergebnisses der Einstellungseinheit (53) in mindestens einer einer Vielzahl der Bildanzeigevorrichtungen die Kompressionsrate der anderen Tomographie-Bilder höher einstellt als die Kompressionsrate des Tomographie-Bildes, das als angezeigt eingestellt ist, und die Vielzahl der Tomographie-Bilder im Speicher (43) speichert.

12. Bildanzeigeverfahren, umfassend:
Anzeigen eines Tomographie-Bildes, für das ein Anzeigebefehl gegeben wird, aus einer Vielzahl von Tomographie-Bildern, die durch Durchführen von Tomosynthese-Bildgebung für ein Objekt erfasst wurden, auf einer Anzeigeeinheit (46);
Einstellen des angezeigten Tomographie-Bildes als angezeigt;
Speichern des Tomographie-Bildes, das als angezeigt eingestellt ist, in einer Speichereinheit (43); und
Speichern anderer Tomographie-Bilder als des Tomographie-Bildes, das als angezeigt eingestellt ist, mit einer Kompressionsrate, die höher ist als die Kompressionsrate des Tomographie-Bildes, das als angezeigt eingestellt ist, oder Löschen anderer Tomographie-Bilder als des Tomographie-Bildes, das als angezeigt eingestellt ist.

13. Bildverwaltungsverfahren, umfassend:
Speichern einer Vielzahl von Tomographie-Bildern, die durch Durchführen von Tomosynthese-Bildgebung für ein Objekt erfasst wurden, in einer Speichereinheit (43), und
Löschen anderer Tomographie-Bilder als des Tomographie-Bildes, das als angezeigt eingestellt ist, aus der Speichereinheit (43) auf Grundlage des Einstellungsergebnisses der Bildanzeigevorrichtung nach Anspruch 1.

14. Bildverwaltungsverfahren, umfassend:
Speichern einer Vielzahl von Tomographie-Bildern, die durch Durchführen von Tomosynthese-Bildgebung für ein Objekt erfasst wurden, in einer Speichereinheit (43); und
Einstellen einer Kompressionsrate anderer Tomographie-Bilder als des Tomographie-Bildes, das als angezeigt eingestellt ist, auf eine höhere Kompressionsrate als die des Tomographie-Bildes, das als angezeigt eingestellt ist, auf Grundlage des Einstellungsergebnisses der Einstellungseinheit (53) in der Bildanzeigevorrichtung nach Anspruch 1, und Speichern der Vielzahl der Tomographie-Bilder in der Speichereinheit (43).

15. Nichtflüchtiges computerlesbares Speichermedium, das ein Bildanzeigeprogramm speichert, das einen Computer veranlasst, Folgendes durchzuführen:
Anzeigen eines Tomographie-Bildes, für das ein Anzeigebefehl gegeben wird, aus einer Vielzahl von Tomographie-Bildern, die durch Durchführen von Tomosynthese-Bildgebung für ein Objekt erfasst wurden, auf einer Anzeigeeinheit (46);
Einstellen des angezeigten Tomographie-Bildes als angezeigt;
Speichern des Tomographie-Bildes, das als angezeigt eingestellt ist, in einer Speichereinheit (43); und
Speichern anderer Tomographie-Bilder als des Tomographie-Bildes, das als angezeigt eingestellt ist, mit einer Kompressionsrate, die höher ist als die Kompressionsrate des Tomographie-Bildes, das als angezeigt eingestellt ist, oder Löschen anderer Tomographie-Bilder als des Tomographie-Bildes, das als angezeigt eingestellt ist.

16. Nichtflüchtiges computerlesbares Speichermedium, das ein Bildverwaltungsprogramm speichert, das den Computer veranlasst, Folgendes durchzuführen:
Speichern einer Vielzahl von Tomographie-Bildern, die durch Durchführen von Tomosynthese-Bildgebung für ein Objekt erfasst wurden, in einer Speichereinheit (43); und
Löschen anderer Tomographie-Bilder als des Tomographie-Bildes, das als angezeigt eingestellt ist, aus der Speichereinheit (43) auf Grundlage des Einstellungsergebnisses der Einstellungseinheit (53) in der Anzeigevorrichtung nach Anspruch 1.

17. Nichtflüchtiges computerlesbares Speichermedium, das ein Bildverwaltungsprogramm speichert, das den Computer veranlasst, Folgendes durchzuführen:
Speichern einer Vielzahl von Tomographie-Bildern, die durch Durchführen von Tomosynthese-Bildgebung für ein Objekt erfasst wurden, in einer Speichereinheit (43); und
Einstellen einer Kompressionsrate anderer Tomographie-Bilder als des Tomographie-Bildes, das als angezeigt eingestellt ist, auf eine höhere Kompressionsrate als die des Tomographie-Bildes, das als angezeigt eingestellt ist, auf Grundlage des Einstellungsergebnisses der Einstellungseinheit (53) in der Bildanzeigevorrichtung nach Anspruch 1, und Speichern der Vielzahl der Tomographie-Bilder in der Speichereinheit (43).

## Revendications

1. Dispositif d'affichage d'image comprenant :
une unité de commande d'affichage (52) qui affiche, sur une unité d'affichage (46), une image tomographique pour laquelle une commande d'affichage est donnée parmi une pluralité d'images tomographiques acquises en effectuant une imagerie de tomosynthèse pour un objet ;
une unité de définition (53) qui définit l'image tomographique affichée comme ayant été affichée ; et
une unité de commande de stockage (54) qui stocke l'image tomographique définie comme ayant été affichée dans une unité de stockage (43), et stocke des images tomographiques autres que l'image tomographique définie comme ayant été affichée à un taux de compression supérieur à un taux de compression de l'image tomographique définie comme ayant été affichée, ou supprime des images tomographiques autres que l'image tomographique définie comme ayant été affichée.

2. Dispositif d'affichage d'image selon la revendication 1, comprenant en outre :
une unité de détection de partie anormale (51) qui détecte une partie anormale à partir de l'image tomographique,
dans lequel l'unité de commande d'affichage (52) affiche une région d'intérêt incluant la partie anormale sur l'unité d'affichage (46) dans le cas où une image tomographique dans laquelle la partie anormale a été détectée par l'unité de détection de partie anormale (51) est affichée.

3. Dispositif d'affichage d'image selon la revendication 1,
dans lequel l'unité de commande d'affichage (46) affiche une image bidimensionnelle composite générée en combinant la pluralité d'images tomographiques sur l'unité d'affichage (46) et affiche une image tomographique incluant une partie anormale désignée dans l'image bidimensionnelle composite sur l'unité d'affichage (46).

4. Dispositif d'affichage d'image selon la revendication 1,
dans lequel, dans le cas où l'imagerie de tomosynthèse est effectuée pour l'objet dans une pluralité de directions et qu'une de la pluralité d'images tomographiques est affichée sur l'unité d'affichage (46) pour l'une quelconque de la pluralité de directions d'imagerie, l'unité de définition (53) définit toutes les images tomographiques dans la direction d'imagerie comme ayant été affichées.

5. Dispositif d'affichage d'image selon la revendication 1,
dans lequel l'unité de définition (53) définit en outre au moins une image tomographique dans un plan tomographique qui est adjacent à l'image tomographique affichée comme ayant été affichée.

6. Dispositif d'affichage d'image selon la revendication 1,
dans lequel l'unité de définition (53) fait passer l'image tomographique définie comme ayant été affichée à un état de non-affichage en réponse à une commande d'un opérateur.

7. Dispositif d'affichage d'image selon la revendication 1,
dans lequel l'objet est une poitrine.

8. Système comprenant :
le dispositif d'affichage d'image selon la revendication 1 ; et
un dispositif de gestion d'image comprenant :
une unité de stockage (43) qui stocke une pluralité d'images tomographiques acquises en effectuant une imagerie de tomosynthèse pour un objet ; et
une unité de commande de stockage (54) qui supprime de l'unité de stockage (43) des images tomographiques autres que l'image tomographique définie comme ayant été affichée sur la base du résultat de définition de l'unité de définition (53) du dispositif d'affichage d'image.

9. Système selon la revendication 8,
dans lequel l'unité de commande de stockage (54) supprime de l'unité de stockage (43) les autres images tomographiques sur la base du résultat de définition de l'unité de définition (53) dans au moins l'un d'une pluralité des dispositifs d'affichage d'image.

10. Système comprenant :
le dispositif d'affichage d'image selon la revendication 1 ; et
un dispositif de gestion d'image comprenant :
une unité de stockage (43) qui stocke une pluralité d'images tomographiques acquises en effectuant une imagerie de tomosynthèse pour un objet ; et
une unité de commande de stockage (54) qui définit un taux de compression d'images tomographiques autres que l'image tomographique définie comme ayant été affichée supérieur à un taux de compression de l'image tomographique définie comme ayant été affichée sur la base du résultat de définition de l'unité de définition (53) du dispositif d'affichage d'image et stocke la pluralité d'images tomographiques dans l'unité de stockage (43).

11. Système selon la revendication 10,
dans lequel l'unité de commande de stockage (54) définit le taux de compression des autres images tomographiques supérieur au taux de compression de l'image tomographique définie comme ayant été affichée sur la base du résultat de définition de l'unité de définition (53) dans au moins l'un d'une pluralité des dispositifs d'affichage d'image et stocke la pluralité d'images tomographiques dans l'unité de stockage (43).

12. Procédé d'affichage d'image comprenant :
l'affichage, sur une unité d'affichage (46), d'une image tomographique pour laquelle une commande d'affichage est donnée parmi une pluralité d'images tomographiques acquises en effectuant une imagerie de tomosynthèse pour un objet ;
la définition de l'image tomographique affichée comme ayant été affichée ;
le stockage de l'image tomographique définie comme ayant été affichée dans une unité de stockage (43) ; et
le stockage d'images tomographiques autres que l'image tomographique définie comme ayant été affichée à un taux de compression supérieur à un taux de compression de l'image tomographique définie comme ayant été affichée, ou la suppression d'images tomographiques autres que l'image tomographique définie comme ayant été affichée.

13. Procédé de gestion d'image comprenant :
le stockage d'une pluralité d'images tomographiques acquises en effectuant une imagerie de tomosynthèse pour un objet dans une unité de stockage (43) et
la suppression de l'unité de stockage (43) d'images tomographiques autres que l'image tomographique définie comme ayant été affichée sur la base du résultat de définition du dispositif d'affichage d'image selon la revendication 1.

14. Procédé de gestion d'image comprenant :
le stockage d'une pluralité d'images tomographiques acquises en effectuant une imagerie de tomosynthèse pour un objet dans une unité de stockage (43) ; et
la définition d'un taux de compression d'images tomographiques autres que l'image tomographique définie comme ayant été affichée supérieur à un taux de compression de l'image tomographique définie comme ayant été affichée sur la base du résultat de définition de l'unité de définition (53) dans le dispositif d'affichage d'image selon la revendication 1 et le stockage de la pluralité d'images tomographiques dans l'unité de stockage (43).

15. Support de stockage non transitoire lisible par ordinateur qui stocke un programme d'affichage d'image qui amène un ordinateur à effectuer :
l'affichage, sur une unité d'affichage (46), d'une image tomographique pour laquelle une commande d'affichage est donnée parmi une pluralité d'images tomographiques acquises en effectuant une imagerie de tomosynthèse pour un objet ;
la définition de l'image tomographique affichée comme ayant été affichée ;
le stockage de l'image tomographique définie comme ayant été affichée dans une unité de stockage (43) ; et
le stockage d'images tomographiques autres que l'image tomographique définie comme ayant été affichée à un taux de compression supérieur à un taux de compression de l'image tomographique définie comme ayant été affichée, ou la suppression d'images tomographiques autres que l'image tomographique définie comme ayant été affichée.

16. Support de stockage non transitoire lisible par ordinateur qui stocke un programme de gestion d'image qui amène un ordinateur à effectuer :
le stockage d'une pluralité d'images tomographiques acquises en effectuant une imagerie de tomosynthèse pour un objet dans une unité de stockage (43) ; et
la suppression de l'unité de stockage (43) d'images tomographiques autres que l'image tomographique définie comme ayant été affichée sur la base du résultat de définition de l'unité de définition (53) dans le dispositif d'affichage selon la revendication 1.

17. Support de stockage non transitoire lisible par ordinateur qui stocke un programme de gestion d'image qui amène un ordinateur à effectuer :
le stockage d'une pluralité d'images tomographiques acquises en effectuant une imagerie de tomosynthèse pour un objet dans une unité de stockage (43) ; et
la définition d'un taux de compression d'images tomographiques autres que l'image tomographique définie comme ayant été affichée supérieur à un taux de compression de l'image tomographique définie comme ayant été affichée sur la base du résultat de définition de l'unité de définition (53) dans le dispositif d'affichage d'image selon la revendication 1 et le stockage de la pluralité d'images tomographiques dans l'unité de stockage (43).
